# EUROPEAN PATENT SPECIFICATION

(11) **EP 3 862 027 B1**
(45) Date of publication and mention of the grant of the patent: **31.12.2025**
(21) Application number: 20305115.6
(22) Date of filing: 07.02.2020
(51) Int. Cl.: A61L 9/03, A61L 9/12, G01N 33/00

(54) **A PORTABLE DEVICE AND METHODS FOR MEASURING DIFFUSION OF FRAGRANCES AND FRAGRANCE RAW MATERIALS**
TRAGBARE VORRICHTUNG UND VERFAHREN ZUR MESSUNG DER DIFFUSION VON DUFTSTOFFEN UND DUFTSTOFFROHMATERIALIEN
DISPOSITIF PORTABLE ET PROCÉDÉS DE MESURE DE LA DIFFUSION DE PARFUMS ET DE MATIÈRES PREMIÈRES DE PARFUMS

(43) Date of publication of application: 11.08.2021
(73) Proprietor: International Flavors & Fragrances Inc., New York, NY 10019 (US)
(72) Inventor: GILLES, Manon, Blandine, Marie, New York 10019 (US); CARPUAT-WEILL, Frederique, Marie-Helene, Brigitte, New York 10019 (US)
(74) Representative: International N&H EMEA

(56) References cited:
- EP-A1- 3 521 823
- WO-A1-2012/093246
- US-A1- 2005 255 008
- US-H- H2 256

## Description

### FIELD OF THE INVENTION

The present disclosure relates to the field of fragrance diffusion and, in particular, to a portable fragrance diffuser useful for measuring diffusion of fragrances or fragrance raw materials in a reproducible and discriminating way, as well as a method of such measurement.

### BACKGROUND OF THE INVENTION

Fragrance diffusers are devices configured to distribute a fragrance throughout an area, and come in multiple sizes, including compact fragrance diffusers for ease of transport. Compact diffusers include thermal vaporizers, which release a predetermined quantity of scent form a liquid reserve by increasing temperature until complete evaporation of the fragrance. The thermal vaporizers do produce fragrances in the form of small droplets. Others compact diffusers that also include a fragrance reservoir are based on the Venturi effect, which results in fragrance atomization via a nozzle leading to small droplets. As the fragrant material evaporates in the compact diffuser, the fragrance is distributed around the area of the diffuser. Sometimes, evaporation is facilitated by high frequency acoustic vibrations that aims to improve diffusion by decreasing fragrance droplet size.

In some diffusers, the fragrance or fragrance ingredient is incorporated into or onto solid supports, such as glass or polymers. Diffusion is then activated by heating the surface and/or by passing an air flow on or through the surface. The air flow gradually drags fragrance molecules from the solid support and diffuses them into the surrounding air.

The existing methods and equipment for the reproduction of a scent are strictly limited in range and quality and do not aim to reproduce consistent fragrance diffusion performance, particularly during movement of a user. See EP 3 521 823 A1, which discloses a method of measuring the spatial-temporal olfactory profile of a perfume composition and a device for generating and emitting a directional air flow, and WO 2017/167407 A1.

US H2256 H discloses a device for detecting human presence by olfactory reception of volatile organic compound molecules dispersed in the air. The device comprises a chamber inlet, a trap, a sensor and a communicator.

WO 2012/093246 discloses a scent dispensing system in which scent impregnated polymer can be heated.

US 2005/0255008 discloses a portable fragrance dispenser including a housing, a fan unit, and an air guide assembly.

For at least these reasons, systems and methods are needed which facilitate adequately measure diffusion of a fragrance in the air from a moving wearer. Fragrance diffusion performance is evaluated as an olfactory intensity assessed by a trained sensory panel or as a quantity of molecules measured analytically by, for example, through Tenax^{©} capture, thermo-desorption, and GC-MS analyses.

At present, the trail often refers to a personal experience of fine fragrance performance when running into someone in the street. However, the challenge for fragrance industry exists in providing a method and a device that allows fragrance or fragrance raw material trail quantification.

### SUMMARY OF THE INVENTION

The present invention is provided to meet the foregoing challenge by providing a portable device, and systems and methods, for measuring fragrance and fragrance raw material trails.

In one aspect, the present invention provides a portable device for measuring fragrance and fragrance raw material diffusions, the device comprising:
a casing;
a diffusion tube coupled to the casing, wherein the diffusion tube has a front end and a rear end, having an opening on each of the front end and the rear end;
a ventilation system housed within the casing;
a heating mat positioned within the casing;
a nozzle;
a sniffing port; and
a sample insertion means,
wherein the diffusion tube is aligned horizontally in relation to the casing,
wherein the opening on the front end of the diffusion tube is plugged into the nozzle, and
wherein the sniffing port is positioned on the rear end of the diffusion tube, characterized in that the casing comprises a bio-sourced polymer and is internally coated with high resistance epoxy resin.

In another aspect, the present invention provides a system for measuring fragrance and fragrance raw material diffusion, the system comprising:
a portable device according to any embodiment disclosed herein;
a wireless High Radio Frequency (HRF) remote controller configured to control one or more components of the portable device; and
a support structure configured to house the portable device and the HRF remote controller.

In another aspect, the present invention provides a method for assessing a fragrance or fragrance raw material trail using a portable device or a system according to any embodiment disclosed herein, wherein the sample insertion means comprises a sample slide and a sample trolley configured to receive the sample slide. The method comprising the following steps:
(i) preparing a fragrance sample or fragrance raw material on the sample slide (deposition and ageing);
(ii) purging the diffusion tube for a period of about 10 to about 30 seconds, optionally using a purge button;
(iii) introducing the fragrance-containing sample into the system through the sample insertion trolley;
(iv) starting the ventilation system to provide a short pulse of air through the diffusion tube;
(v) performing olfactory intensity and/or hedonic assessment by a trained sensory panel;
(vi) connecting sniffing port screw caps adaptor to an analytical instrument (e.g., Tenax^{©} capture, thermo-desorption, and GC-MS) measure quantity of the fragrance or fragrance raw material molecules; and
(vii) analyzing data.

Other aspects and advantages of the present invention will be better appreciated in view of the following drawings, detailed description, and claims.

### BRIEF DESCRIPTION OF THE DRAWINGS

FIG. 1 illustrates a perspective view of a system to measure fragrances and fragrance raw materials diffusion, in accordance with various embodiments of the present disclosure.
FIG. 2 illustrates in an exploded view of an example of a device to measure fragrances and fragrance raw materials diffusion, in accordance with various embodiments of the present disclosure.
FIG. 3 illustrates in a 3-dimensional view an example of a system diffusion device and tube, in accordance with various embodiments of the present disclosure.
FIG. 4 illustrates an example of a High Radio Frequency (HRF) remote controller in an exploded view, in accordance with various embodiments of the present disclosure.
FIG. 5 illustrates in a perspective view of an example of a device support, in accordance with various embodiments of the present disclosure.
FIG. 6 illustrates a flow chart of a method for assessing fragrance or fragrance raw materials trail, according to various embodiments of the present disclosure.

### DETAILED DESCRIPTION OF THE INVENTION

In one aspect, the present invention provides a portable device for measuring fragrance and fragrance raw material diffusions, the device comprising:
a casing;
a diffusion tube coupled to the casing, wherein the diffusion tube has a front end and a rear end, having an opening on each of the front end and the rear end;
a ventilation system housed within the casing;
a heating mat positioned within the casing;
a nozzle;
a sniffing port; and
a sample insertion means,
wherein the diffusion tube is aligned horizontally in relation to the casing,
wherein the opening on the front end of the diffusion tube is plugged into the nozzle, and
wherein the sniffing port is positioned on the rear end of the diffusion tube,
characterized in that the casing comprises a bio-sourced polymer and is internally coated with high-resistance epoxy resin.

In one embodiment, the sample insertion means in the portable device comprises a sample slide and a sample trolley configured to receive the sample slide; wherein the sample slide is optionally made from a material selected from the group consisting of glass, polymer, ceramic, metal, fabric, paper, artificial skin, hair swatch, and combinations thereof.

In another embodiment, the casing of the portable device is made from a material selected from the group consisting of polylactic acid, polyhydroxyalkanoates, and combinations thereof; and wherein optionally the casing is fully detachable and washable.

In some embodiments, the casing is made from a material selected from the group consisting of polylactic acid, polyhydroxyalkanoates, cellulose acetate, starch blends, and combinations thereof; and wherein optionally the casing is fully detachable and washable.

In another embodiment, the casing of the portable device is in a parallelepiped form having a width between about 15 cm and about 25 cm, a depth between about 5 cm and about 15 cm, and a height between about 5 cm and about 15 cm.

In another embodiment, the diffusion tube of the portable device is made from a material selected from the group consisting of glass, metal, plastic, polymer, bio-sourced polymer, and combinations thereof, and is optionally coated with epoxy resin.

In another embodiment, the diffusion tube of the portable device is made from aluminum and coated with epoxy resin.

In another embodiment, the diffusion tube of the portable device has a length between about 50 cm and about 150 cm and a section diameter between about 5 cm and about 10 cm.

In another embodiment, the diffusion tube of the portable device is equipped with a sniffing port located in the rear end of the diffusion tube.

In another embodiment, the sniffing port of the portable device comprises a component selected from the group consisting of sensors, screw caps, and fittings configured to adapt one or more analytical instruments.

In another embodiment, the ventilation system of the portable device is configured to generate an air pulse.

In another embodiment, the air pulse lasts between 1 second and 5 seconds; and optionally the air pulse has an air flow speed between about 1 km/h and about 15 km/h.

In another aspect, the present invention provides a system for measuring fragrance and fragrance raw material diffusion, the system comprising:
a portable device according to any embodiment disclosed herein;
a wireless High Radio Frequency (HRF) remote controller configured to control one or more components of the portable device; and
a support structure configured to house the portable device and the HRF remote controller.

In another aspect, the present invention provides a method for assessing a fragrance or fragrance raw material trail using a system according to any embodiment disclosed herein, the method comprising the following steps:
(i) preparing a fragrance sample or fragrance raw material on the sample slide (deposition and ageing);
(ii) purging the diffusion tube for a period of about 10 to about 30 seconds, optionally using a purge button;
(iii) introducing the fragrance-containing sample into the system through the sample insertion trolley;
(iv) starting the ventilation system to provide a short pulse of air through the diffusion tube;
(v) performing olfactory intensity and/or hedonic assessment by a trained sensory panel;
(vi) connecting sniffing port screw caps adaptor to an analytical instrument (e.g., Tenax^{©} capture, thermo-desorption, and GC-MS) measure quantity of the fragrance or fragrance raw material molecules; and
(vii) analyzing data.

In one embodiment, in the method disclosed herein, about 5 µL to about 100 µL of fragrance or fragrance raw material is deposited on the sample slide for assessment.

In one embodiment, in the method disclosed herein, the fragrance sample slide can be heated to about 25 °C to about 35 °C before or during olfactory evaluation.

As used in this document, the singular forms "a," "an," and "the" include plural references unless the context clearly dictates otherwise. Unless defined otherwise, all technical and scientific terms used herein have the same meanings as commonly understood by one of ordinary skill in the art. When used in this document, the term "comprising" (or "comprises") means "including (or includes), but not limited to." When used in this document, the term "exemplary" is intended to mean "by way of example" and is not intended to indicate that a particular exemplary item is preferred or required.

In this document, when terms such "first" and "second" are used to modify a noun, such use is simply intended to distinguish one item from another, and is not intended to require a sequential order unless specifically stated. The term "approximately," when used in connection with a numeric value, is intended to include values that are close to, but not exactly, the number. For example, in some embodiments, the term "approximately" may include values that are within +/- 10 percent of the value.

As used herein, the term "about" generally includes up to plus or minus 10% of the indicated number. For example, "about 10%" may indicate a range of 9% to 11%, and "about 20" may mean from 18 to 22. Sometimes preferably, the term "about" includes up to plus or minus 5% of the indicated value. As disclosed herein, a number of ranges of numeric values are provided. It is understood that each intervening value, to the tenth of the unit of the lower limit, unless the context clearly dictates otherwise, between the upper and lower limits of that range is also specifically disclosed.

A "portable fragrance diffuser", as used herein, refers to a light diffuser of small overall size that can be held in one hand, or an overall system (diffuser, tube(s), support and consumables) that can be transported in a specifically-designed suitcase.

Other terms that are relevant to this disclosure are defined at the end of this Detailed Description section.

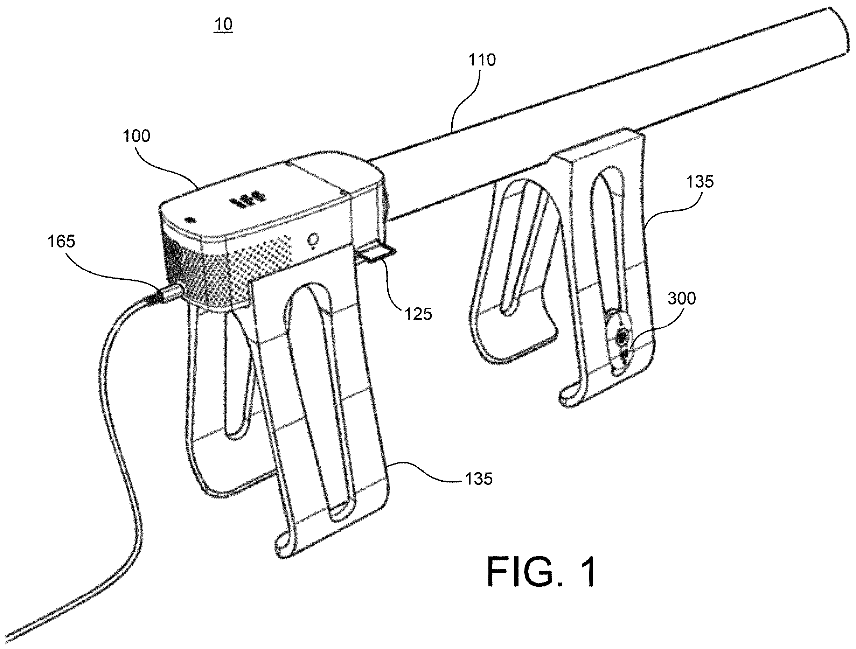

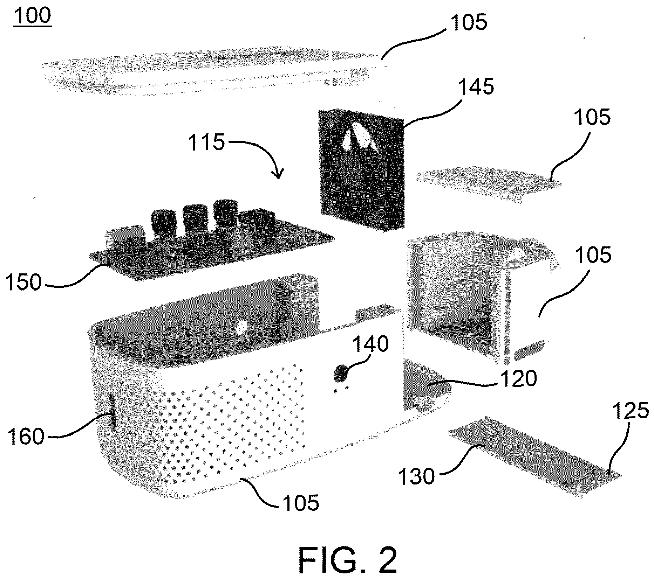

Referring now to FIG. 1, a system 10 and FIG. 2, a portable device 100 (also shown in FIG. 1) for measuring fragrances and fragrance raw materials diffusion is illustratively depicted, in accordance with various embodiments of the present disclosure. According to various embodiments, the portable device 100 is configured to enable the measuring of one or more fragrances of a fragrance raw material trail, and also provides a means for assessing fragrance diffusion using analytical means and/or sensory evaluations by a trained panel.

According to various embodiments, the portable device 100 includes a casing 105, including one or more movable components. The casing 105 may be produced by 3-dimensional (3D) printing techniques, via molding techniques, and/or via any other suitable form of production. According to various embodiments, the casing 105 includes a bio-sourced polymer and is internally coated with high resistance epoxy resin and, preferably, includes polylactic acid (PLA), polyhydroxy alkanoate (PHA). Exemplary bio-sourced polymers, are listed in Table 1.

**Table 1. Examples of suitable polymers**

| Polymer | Abbreviation | Average Biomass Content of Polymer |
|---|---|---|
| Cellulose Acetate | CA | 50% |
| Polyhydroxy Alkanoate | PHAs | 100% |
| Polylactic Acid | PLA | 100% |
| Starch Blends | | 40% |

According to various embodiments, the casing **105** is in the general form of a parallelepiped of approximately 15 cm to 25 cm wide, approximately 5 cm to 15 cm deep, and approximately 5 cm to 15 cm high. It is noted, however, that other suitable dimensions for the casing **105** may be incorporated. According to various embodiments, the casing **105** including multiple components (as shown in FIG. 2), which are configured to be easily and readily disassembled and washed to avoid cross contamination.

The portable device **100** may include one or more power switches **160** configured to turn one or more electronic components of the portable device **100** on or off. According to an embodiment, the one or more power switches **160** includes a button. It is noted, however, that other suitable forms of power switches **160** may, alternatively or in addition, be incorporated. According to various embodiments, the device **100** is powered by an internal power supply and/or an external power source **165.**

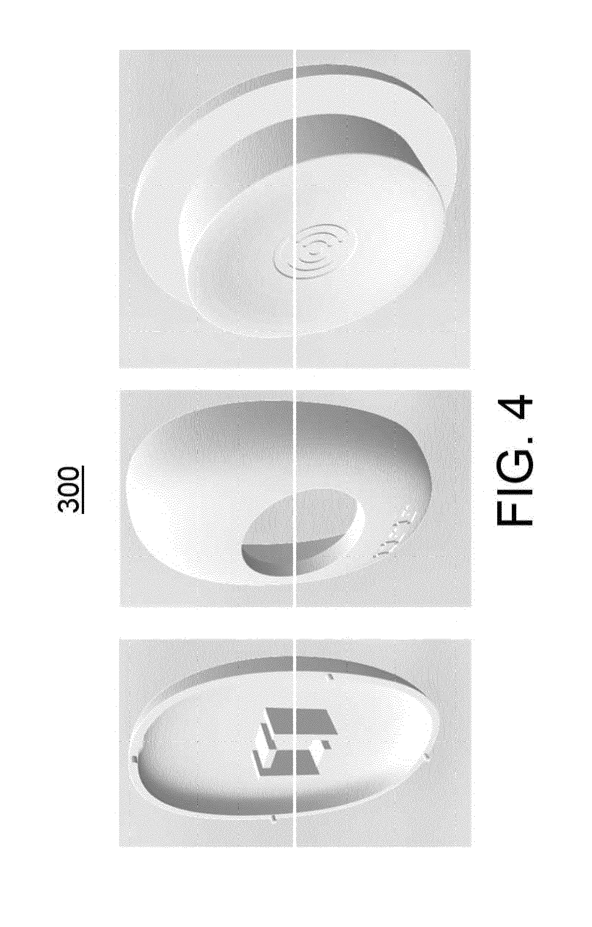

The portable device **100** may include a controllable and stable pulse ventilation system **115.** According to various embodiments, the ventilation system **115** includes a fan **145.** The ventilation system **115** may further include a printed circuit board **150** configured to regulate the speed of the fan **145.** The air flow speed is stable and is configured to be set to a stable and precise speed between approximately 1 km/h and approximately 15 km/h. According to various embodiments, the ventilation system **115** is pulsed, and its duration is set at a value between approximately 1 second and approximately 5 seconds. According to various embodiments, pulsed air flow is controllable by a user through a High Radio Frequency (HRF) remote controller **300** (as shown in FIGs. 1 and 4). According to various alternative embodiments, continuous **airflow** of the same speed as the pulse is generated and can be activated to purge the portable device **100** and the diffusion tube **110** through a button **155** on the casing **105.**

The portable device **100** may include a heated mat **120.** The heated mat **120** may include, for example, silicone and/or any other suitable materials. According to various embodiments, the temperature of the heated mat **120** may be activated and/or disengaged through a button **140** on the casing **105.** According to various embodiments, a heating temperature produced by the heated mat **120** may be approximately 31°C to approximately 33°C. It is noted, however, that other temperatures may be incorporated, while maintaining the spirit of the present disclosure. According to various embodiments, an indicator LED indicates when the set temperature is reached.

According to various embodiments, the portable device **100** is configured to receive one or more fragrance samples which may be inserted into the portable device **100** via, for example, a sample insertion trolley **125** and a sample slide **130.** It is noted, however, that any suitable form of sample insertion may be incorporated, while maintaining the spirit of the present disclosure. According to various embodiments, the sample slide **130** is inserted into the casing **105** via the sample insertion trolley **125.** According to various embodiments, the sample slide **130 is** glass. It is noted, however, that any suitable material (for example, glass, polymers, ceramic, metal, fabric, paper, artificial skin, and combinations thereof) for the sample slide **130** may be used, while maintaining the spirit of the present disclosure. Any other material that aims to mimic fragrance diffusion from a fragranced support may be incorporated. The sample insertion trolley **125** may be configured to avoid contact between the casing **105** and an upper surface of the sample plate **130** during its insertion. The sample insertion trolley **125** may be positioned onto the heated mat **120** whose heating function can be activated or not through a button **140** on the casing **105.** According to various embodiments, samples may be introduced without the sample insertion trolley **125** by positioning the sample slide **130** directly into the portable device **100.** According to various embodiments, approximately 5 µL to approximately 100 µL of fragrance or of fragrance ingredient sample is deposited on a sample slide **130** of approximately 75 x 25 mm. It is noted, however, that other sample slide **130** dimensions may be incorporated, which may be capable of having deposited more or less fragrance. Alternatively, fragranced samples are directly inserted into the portable device, without using the sample slide.

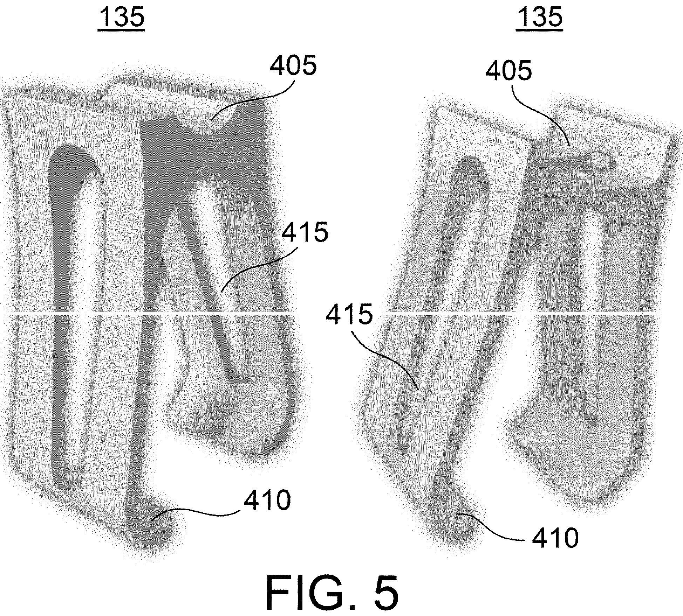

The portable device **100** may further include a support structure **135** (as shown in FIG. 5). The support structure **135** may be produced using 3D printing techniques, via molding techniques, and/or via any other suitable form of production. According to various embodiments, the casing **105** is configured to be mounted onto a receiving portion **405** of the support structure. According to various embodiments, the support structure **135** includes one or more legs **410.** The one or more legs **410** may have a height of approximately 20 cm to approximately 40 cm. It is noted, however, that other heights may alternatively be employed, while maintaining the spirit of the present disclosure. According to various embodiments, the support structure **135** includes an opening **415** configured to store the HRF remote controller **300** (as shown in FIGs. 1 and 4).

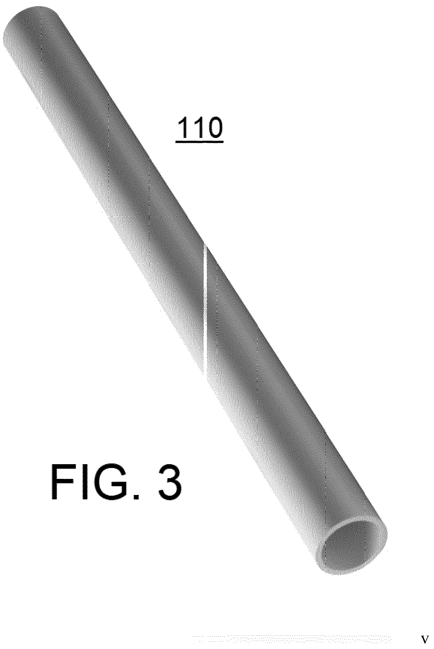

The portable device **100** may include one or more diffusion tube **110.** Referring now to FIG. 3, a diffusion tube **110** is illustratively depicted, in accordance with various embodiments of the present disclosure.

The diffusion tube **110** may be opened on both ends. The diffusion tube **110** may be positioned horizontally in relation to the casing **105.** The diffusion tube **105** may include a front opening and a rear opening. According to various embodiments, the front opening of the diffusion tube **110** is configured to be plugged into the nozzle of the casing **105.** According to various embodiments, the diffusion tube **110** includes one or more sniffing ports. The sniffing port may be positioned on a rear section of the diffusion tube **110.**

According to various embodiments, the fragrance diffusion tube **110** includes an aluminum cylinder internally coated with high resistance epoxy resin. It is noted, however, that other materials such as, for example, glass, plastic, and/or any other materials that may be coated with any resin or polymer that show low interaction with Volatile Organic Compounds (VOCs), may be included in the diffusion tube **110.** The diffusion tube **110** may be opened on both ends. According to various embodiment, the diffusion tube **110** may be aligned horizontally and displayed on the sniffing port on the rear section of the diffusion tube **110.**

According to an alternative embodiment, the sniffing port is positioned at a rear section of the diffusion tube **110,** in front of the airflow. This sniffing port may alternatively be used to adapt analytical instruments (for example, Tenax^{©} tube and pump or SPME fibers followed by GC-MS analyses, sensors, etc.). The front opening of the diffusion tube **110** may be plugged on the casing **105** nozzle that includes the ventilation system **115** that delivers a controlled pulsed air flow into the diffusion tube **110.**

The front of the diffusion tube **110** refers to the part of the diffusion tube **110** starting from the opening plugged on the casing **105** where ventilator and sample are located. The rear of the diffusion tube **110** refers to the second opening at the end of the diffusion tube, where sniffing port, sensor or analytical instruments can be adapted. The diffusion tube **110** may have a length of approximately 50 cm to approximately 150 cm, and a diameter of approximately 5 cm to approximately 10 cm. It is noted, however, that other suitable lengths may be incorporated, while maintaining the spirit of the present disclosure.

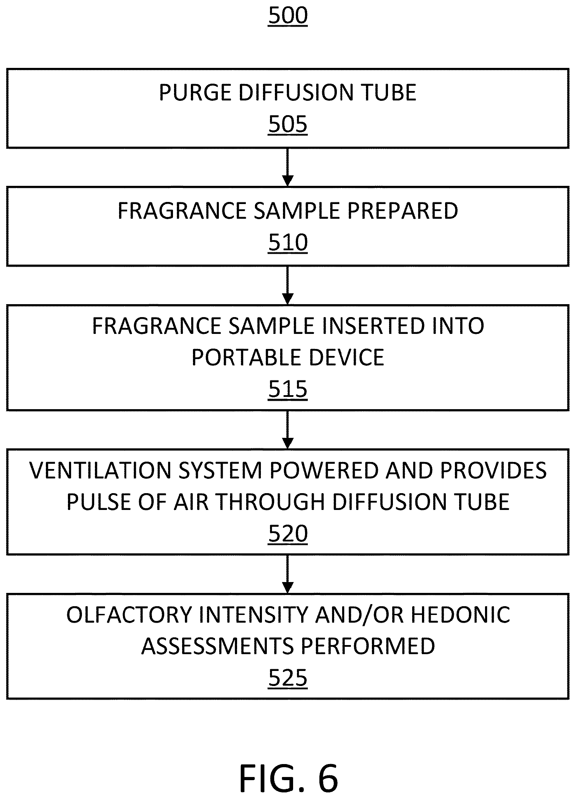

Referring now to FIG. 6, a method **500** for assessing fragrance or fragrance raw materials trails is illustratively depicted, in accordance with the present disclosure.

At **505,** the diffusion tube is purged. According to various embodiments, the purging lasts approximately 10 seconds to 30 seconds. It is noted, however, that any suitable timeframe for purging may be incorporated. Once purged, at **510,** a fragrance sample is prepared on a sample slide and, at **515,** the sample positioned on the slide is inserted into the portable device, as described in FIGs. 1-2.

Once the sample is inserted into the portable device, the ventilation system, at **520,** is powered and provides a short pulse of air through the diffusion tube. Once the air is passed through the diffusion tube, one or more olfactory intensity and/or hedonic assessments, at **525,** are performed by a trained sensory panel. Screw caps, at **530,** are connected to measure quantities of molecules in the sample analytically and, at **535,** measured data is analyzed.

The features and functions described above, as well as alternatives, may be combined into many other different systems or applications. Various alternatives, modifications, variations or improvements may be made by those skilled in the art, each of which is also intended to be encompassed by the disclosed embodiments.

The device and systems disclosed herein are used to assess fine fragrances that are on the market, which include, but are not limited to, the following fragrances under the trademarks of YSL Paris^{®} by Yves Saint Laurent (YSL), Dior^{®} J'adore, YSL Mon Paris^{®}, Gabrielle Chanel^{®}, La Vie est Belle^{®} by Lancôme, Alien^{®} by Thierry Mugler, Idole^{®} by Lancôme, Miss Dior Blooming Bouquet^{®} by Dior, Black Opium^{®} by YSL, Chance by Chanel, Flowerbomb^{®} by Victor & Rolf, Bleu de Chanel^{®}, La nuit de L'homme^{®} by YSL, Emblem^{®} by Mont Blanc, Invictus^{®} by Paco Rabanne, One Million by Paco Rabanne, Sauvage^{®} by Dior, Only the Brave^{®} by Diesel, and Wanted^{®} by Azarro, and the like.

The device and systems are also applicable to assess any fragrance raw materials (either natural or synthetic), preferably diluted in ethanol at a concentration depending on its impact (for example, using 1% w/w as a standard concentration), such as 2,4-dimethyl-3-cyclohexenecarboxaldehyde, 2,6-dimethyloct-7-en-2-ol, (3-oxo-2-pentylcyclopentyl)acetic acid methyl ester, 1-(1,2,3,4,5,6,7,8-octahydro-2,3,8,8,-tetramethyl-2-naphthyl)ethan-1-one, 2-phenylethan-1-ol, 4-hydroxyl-3-methoxybenzaldehyde, 2-methoxy-4-(2-propenyl)-phenol, (3aR,5aS,9aS,9bR)-3a,6,6,9a-tetramethyl-2,4,5,5a,7,8,9,9b-octahydro-1H-benzo[e][1]benzofuran, 3,7-dimethylocta-1,6-dien-3-ol, methyl 2,4-dihydroxy-3,6-dimethylbenzoate, benzyl acetate, 4-(4-hydroxyphenyl)-butan-2-one, 5-hexyloxolan-2-one, 2-ethyl-3-hydroxy-4-pyranone, 3,7-dimethylocta-1,6-dien-3-yle acetate, 2H-1-benzopyrane-2-one, patchouli oil, bergamot oil, oud oil, cedarwood oil, Jasmin absolute, and the like.

The device and systems are also applicable to assessing any commercial products containing fragrances or fragrance raw materials, such as an Eau de Toilette product, an Eau de Parfum, a cologne, a scent booster, an encapsulated fragrance, a fine fragrance, a men's fine fragrance, a women's fine fragrance, a perfume, a solid perfume, a natural spray product, a perfume spray product, a home care product, a reed diffuser, an all-purpose cleaner, a floor cleaner, a toilet cleaner, a toilet rimblock, a bath tissue, liquid air freshener, air freshener spray, a spray dispenser product, an incense stick, a rug deodorizer, a candle, a room deodorizer, a liquid dish detergent, a paste dish detergent, an anti-inflammatory balm, an anti-inflammatory ointment, an anti-inflammatory spray, a disinfectant, a personal care product, a soap, a bar soap, a liquid soap, a bath fragrance, a body wash, a non-aerosol body spray, a body milk, a cleanser, a body cream, a hand sanitizer, a hand wash, a functional product base, a sunscreen lotion, a sunscreen spray, a deodorant, an anti-perspirant, an roll-on product, an aerosol product, a natural spray product, a wax-based deodorant, a glycol type deodorant, a soap type deodorant, a facial lotion, a body lotion, a hand lotion, a miscellaneous lotion, a body powder, a shave cream, a shave gel, a shave butter, a bath soak, a shower gel, an exfoliating scrub, a foot cream, a facial tissue, a cleansing wipe, a talc product, a hair care product, a hair care with ammonia, a shampoo, a hair conditioner, a hair rinse, a hair refresher, a hair fixative or styling aid, a hair bleach, a hair dye or colorant, a fabric care product, a fabric softener, a liquid fabric softener, a fabric softener sheet, a drier sheet, a fabric refresher, an ironing water, a detergent, a laundry detergent, a liquid laundry detergent, a powder laundry detergent, a tablet laundry detergent, a laundry detergent bar, a laundry detergent cream, a hand wash laundry detergent, an insect repellent product, a wildlife scent, and the like.

### EXAMPLE 1: Measurement of Fragrance Intensity

A portable device of this invention was assembled in the facility operated by Tweener (Wattignies, France).

Nineteen fragrances were tested including 11 feminine fragrances and 8 masculine fragrances. Each fragrance was applied on a glass slide for evaluation. Fragrance intensity was rated both just after application (time 0) and after 4 hours sitting at 32°C (Time 4 hr). The fragrance intensity was rated at a scale ranging from 0 to 10. A numerical value of 2 indicates that the composition produces a weak intensity. A value of 10 indicates that the composition generates a very strong smell. Evaluation is performed by a trained sensory panel. The sensory results are given in Table 2. The results indicated that the device is efficient in ranking fragrances and differentiating their levels of diffusion.

**Table 2: Fragrance intensity**

| | **Fragrance** | **Intensity Time 0** | **Intensity Time 4 hr** |
|---|---|---|---|
| Intensity | Feminine Fragrance 1 | 7.2 | 6 |
| | Feminine Fragrance 2 | 6.8 | 5.7 |
| | Feminine Fragrance 3 | 6.8 | 6 |
| | Feminine Fragrance 4 | 6.8 | 6.1 |
| | Feminine Fragrance 5 | 6.8 | 6.1 |
| Feminine Fragrance 6 | | 6.7 | 6.7 |
| Feminine Fragrance 7 | | 6.7 | 6.1 |
| Feminine Fragrance 8 | | 6.5 | 6.1 |
| Feminine Fragrance 9 | | 6.4 | 5.8 |
| Feminine Fragrance 10 | | 6.4 | 6.1 |
| Feminine Fragrance 11 | | 5.9 | 6 |
| Masculine Fragrance 1 | | 6.8 | 5.9 |
| Masculine Fragrance 2 | | 6.8 | 6 |
| Masculine Fragrance 3 | | 6.7 | 6.2 |
| Masculine Fragrance 4 | | 6.6 | 5.5 |
| Masculine Fragrance 5 | | 6.6 | 5.9 |
| Masculine Fragrance 6 | | 6.5 | 5.9 |
| Masculine Fragrance 7 | | 6.5 | 6.2 |
| Masculine Fragrance 8 | | 6.2 | 5.7 |

### EXAMPLE 2: Measurement reproducibility

To establish the reproducibility of the measurement using the device described in Example 1, four fragrances were tested repeatedly. Each fragrance was applied on a glass slide for evaluation. Fragrance diffusion intensity was rated both just after application (Time 0) and after 4hr aging at 32°C (Time 4 hr). The fragrance intensity was rated at a scale ranging from 0 to 10. A numerical value of 2 indicates that the composition produces a weak intensity. A value of 10 indicates that the composition generates a very strong smell. Evaluation is performed by a trained sensory panel in two different sessions. The sensory results are given in Table 3. The analysis indicated that the device gives reliable and reproducible diffusion data.

**Table 3: Diffusion measurements reproducibility**

| **Fragrance** | **1^{st} measurement Time 0** | **2^{nd} Measurement Time 0** | **1^{st} measurement Time 4 hr** | **2^{nd} Measurement Time 4 hr** |
|---|---|---|---|---|
| Fragrance 1 | 7.1 | 7.2 | 6 | 6 |
| Fragrance 2 | 6.7 | 6.7 | 6.6 | 6.7 |
| Fragrance 3 | 6.7 | 6.7 | 6 | 6.2 |
| Fragrance 4 | 6.4 | 5.9 | 6 | 6 |

## Claims

1. A portable device (100) for measuring fragrance and fragrance raw material diffusion, comprising:
a casing (105);
a diffusion tube (110) coupled to the casing, wherein the diffusion tube has a front end and a rear end, having an opening on each of the front end and the rear end;
a ventilation system (115) housed within the casing;
a mat (120) positioned within the casing, wherein the mat can be heated;
a nozzle;
a sniffing port; and
a sample insertion means,
wherein the diffusion tube is aligned horizontally in relation to the casing,
wherein the opening on the front end of the diffusion tube is plugged into the nozzle,
wherein the sniffing port is positioned on the rear end of the diffusion tube,
**characterized in that** the casing comprises a bio-sourced polymer and is internally coated with high resistance epoxy resin.

2. The portable device of claim 1, wherein the sample insertion means comprises a sample slide (130) and a sample trolley (125) configured to receive the sample slide; wherein the sample slide is optionally made from a material selected from the group consisting of glass, polymer, ceramic, metal, fabric, paper, artificial skin, hair swatch, and combinations thereof.

3. The portable device of claim 1 or 2, wherein the bio-sourced polymer is selected from the group consisting of polylactic acid, polyhydroxyalkanoates, cellulose acetate, starch blends, and combinations thereof; and wherein optionally the casing is fully detachable and washable.

4. The portable device of any one of claims 1 to 3, wherein the casing is in a parallelepiped form having a width between about 15 cm and about 25 cm, a depth between about 5 cm and about 15 cm, and a height between about 5 cm and about 15 cm.

5. The portable device of any one of claims 1 to 4, wherein said diffusion tube is made from a material selected from the group consisting of glass, metal, plastic, polymer, bio-sourced polymer, and combinations thereof, and is optionally coated with epoxy resin.

6. The portable device of any one of claims 1 to 5, wherein said diffusion tube is made from aluminum and coated with epoxy resin.

7. The portable device of any one of claims 1 to 6, wherein said diffusion tube has a length between about 50 cm and about 150 cm and a section diameter between about 5 cm and about 10 cm.

8. The portable device of any one of claims 1 to 7, wherein the diffusion tube is equipped with a sniffing port located in the rear end of the diffusion tube.

9. The portable device of any one of claims 1 to 8, wherein the sniffing port comprises a component selected from the group consisting of sensors, screw caps, and fittings configured to adapt one or more analytic instruments.

10. The portable device of claim 9, wherein the ventilation system is configured to generate an air pulse; and optionally wherein the air pulse lasts between 1 second and 5 seconds; and optionally wherein the air pulse has an air flow speed between about 1 km/h and about 15 km/h.

11. A system (10) for measuring fragrance and fragrance raw material diffusion, comprising:
a portable device of any one of claims 1 to 10;
a wireless High Radio Frequency (HRF) remote controller (300) configured to control one or more components of the portable device; and
a support structure (135) configured to house the portable device and the HRF remote controller.

12. A method (500) for assessing the fragrance or fragrance raw material trail using a portable device of any one of claims 1 to 10 or the system of claim 11 wherein the sample insertion means comprises a sample slide (130) and a sample trolley (125) configured to receive the sample slide, the method comprising the following steps:
(i) preparing (510) a fragrance or fragrance raw material sample on the sample slide;
(ii) purging (505) the diffusion tube for a period of about 10 to about 30 seconds, optionally using a purge button (155);
(iii) introducing (515) the fragrance-containing sample slide into the system through the sample insertion trolley;
(iv) starting (520) the ventilation system to provide a short pulse of air through the diffusion tube;
(v) performing (525) olfactory intensity and/or hedonic assessment by a trained sensory panel;
(vi) connecting (530) sniffing port screw caps adaptor to an analytical instrument measure quantity of the fragrance or fragrance raw material molecules; and
(vii) analyzing data (535).

13. The method of Claim 12, wherein about 5 µL to about 100 µL of fragrance or fragrance raw material is deposited on the sample slide for assessment.

14. The method of any of claims 12 or 13, further comprising heating the sample to about 25 °C to about 35 °C before or during olfactory evaluation.

## Patentansprüche

1. Tragbare Vorrichtung (100) zum Messen von Duftstoff- und Duftstoffausgangsmaterialdiffusion, umfassend:
ein Gehäuse (105);
ein Diffusionsrohr (110), das an das Gehäuse gekoppelt ist, wobei das Diffusionsrohr ein vorderes Ende und ein hinteres Ende aufweist, die jeweils eine Öffnung an dem vorderen Ende bzw. dem hinteren Ende aufweisen;
ein Lüftungssystem (115), das in dem Gehäuse untergebracht ist;
eine Matte (120), die in dem Gehäuse angeordnet ist, wobei die Matte erhitzt werden kann;
eine Düse;
eine Riechöffnung; und
ein Probeneinführmittel,
wobei das Diffusionsrohr horizontal bezogen auf das Gehäuse ausgerichtet ist,
wobei die Öffnung an dem vorderen Ende des Diffusionsrohrs in die Düse eingesteckt ist,
wobei die Riechöffnung an dem hinteren Ende des Diffusionsrohrs angeordnet ist,
**dadurch gekennzeichnet, dass** das Gehäuse aus einem Polymer auf Biobasis besteht und an der Innenseite mit hochfestem Epoxyharz beschichtet ist.

2. Tragbare Vorrichtung nach Anspruch 1, wobei das Probeneinführmittel einen Probenträger (130) und einen Probenschlitten (125), der zum Aufnehmen des Probenträgers gestaltet ist, umfasst; wobei der Probenträger gegebenenfalls aus einem Material ausgewählt aus der Gruppe bestehend aus Glas, Polymer, Keramik, Metall, Gewebe, Papier, künstlicher Haut, Haarmuster und Kombinationen davon besteht.

3. Tragbare Vorrichtung nach Anspruch 1 oder 2, wobei das Polymer auf Biobasis ausgewählt ist aus der Gruppe bestehend aus Polymilchsäure, Polyhydroxyalkanoaten, Celluloseacetat, Stärkegemischen und Kombinationen davon; und wobei gegebenenfalls das Gehäuse vollständig abnehmbar und waschbar ist.

4. Tragbare Vorrichtung nach einem der Ansprüche 1 bis 3, wobei das Gehäuse in einer Parallelepipedform mit einer Breite zwischen etwa 15 cm und etwa 25 cm, einer Tiefe zwischen etwa 5 cm und etwa 15 cm und einer Höhe zwischen etwa 5 cm und etwa 15 cm vorliegt.

5. Tragbare Vorrichtung nach einem der Ansprüche 1 bis 4, wobei das Diffusionsrohr aus einem Material ausgewählt aus der Gruppe bestehend aus Glas, Metall, Kunststoff, Polymer, Polymer auf Biobasis und Kombinationen davon besteht und gegebenenfalls mit Epoxyharz beschichtet ist.

6. Tragbare Vorrichtung nach einem der Ansprüche 1 bis 5, wobei das Diffusionsrohr aus Aluminium besteht und mit Epoxyharz beschichtet ist.

7. Tragbare Vorrichtung nach einem der Ansprüche 1 bis 6, wobei das Diffusionsrohr eine Länge zwischen etwa 50 cm und etwa 150 cm und einen Querschnittsdurchmesser zwischen etwa 5 cm und etwa 10 cm aufweist.

8. Tragbare Vorrichtung nach einem der Ansprüche 1 bis 7, wobei das Diffusionsrohr mit einer Riechöffnung versehen ist, die an dem hinteren Ende des Diffusionsrohrs angeordnet ist.

9. Tragbare Vorrichtung nach einem der Ansprüche 1 bis 8, wobei die Riechöffnung eine Komponente ausgewählt aus der Gruppe bestehend aus Sensoren, Schraubkappen und Anschlussstücken, die zum Passen an ein oder mehrere analytische Geräte gestaltet sind, umfasst.

10. Tragbare Vorrichtung nach Anspruch 9, wobei das Lüftungssystem dafür gestaltet ist, einen Luftimpuls zu erzeugen; und wobei gegebenenfalls der Luftimpuls zwischen 1 Sekunde und 5 Sekunden dauert; und wobei gegebenenfalls der Luftimpuls eine Luftströmungsgeschwindigkeit zwischen etwa 1 km/h und etwa 15 km/h aufweist.

11. System (10) zum Messen von Duftstoff- und Duftstoffausgangsmaterialdiffusion, umfassend:
eine tragbare Vorrichtung nach einem der Ansprüche 1 bis 10;
eine drahtlose Hochfrequenz(HRF)-Fernsteuerung (300), dafür gestaltet, eine oder mehrere Komponenten der tragbaren Vorrichtung zu steuern; und
eine Trägerstruktur (135), dafür gestaltet, die tragbare Vorrichtung und die HRF-Fernsteuerung aufzunehmen.

12. Verfahren (500) zum Beurteilen der Duftstoff- oder Duftstoffausgangsmaterial-Spur unter Verwendung einer tragbaren Vorrichtung nach einem der Ansprüche 1 bis 10 oder des Systems nach Anspruch 11, wobei das Probeneinführmittel einen Probenträger (130) und einen Probenschlitten (125), der zum Aufnehmen des Progenträgers getsaltet ist, umfasst, wobei das Verfahren die folgenden Schritte umfasst:
(i) Vorbereiten (510) einer Duftstoff- oder Duftstoffausgangsmaterial-Probe auf dem Probenträger;
(ii) Spülen (505) des Diffusionsrohrs für einen Zeitraum von etwa 10 bis etwa 30 Sekunden, gegebenenfalls unter Verwendung eines Spülknopfs (155);
(iii) Einführen (515) des Duftstoff-enthaltenden Probenträgers in das System durch den Probeneinführschlitten;
(iv) Starten (520) des Lüftungssystems, um einen kurzen Luftimpuls durch das Diffusionsrohr zu erzeugen;
(v) Durchführen (525) einer olfaktorischen Intensitäts- und/oder hedonischen Bewertung durch ein geschultes Sensorikpanel;
(vi) Verbinden (530) eines Riechöffnungs-Schraubkappenadapters an ein Analysegerät zum Messen der Menge der Duftstoff- oder Duftstoffausgangsmaterial-Moleküle; und
(vii) Analysieren von Daten (535).

13. Verfahren nach Anspruch 12, wobei etwa 5 µl bis etwa 100 µl Duftstoff- oder Duftstoffausgangsmaterial zur Beurteilung auf dem Probenträger aufgebracht werden.

14. Verfahren nach einem der Ansprüche 12 oder 13, ferner umfassend Erwärmen der Probe auf etwa 25 °C bis etwa 35 °C vor oder während der olfaktorischen Bewertung.

## Revendications

1. Dispositif portable (100) de mesure de la diffusion de parfums et de matières premières de parfums, comprenant :
un boîtier (105) ;
un tube de diffusion (110) accouplé au boîtier, le tube de diffusion ayant une extrémité avant et une extrémité arrière, ayant une ouverture sur chacune de l'extrémité avant et de l'extrémité arrière ;
un système de ventilation (115) logé à l'intérieur du boîtier ;
un tapis (120) positionné à l'intérieur du boîtier, le tapis pouvant être chauffé ;
une buse ;
un orifice d'échantillonnage olfactif ; et
un moyen d'insertion d'échantillon,
le tube de diffusion étant aligné horizontalement par rapport au boîtier,
l'ouverture sur l'extrémité avant du tube de diffusion étant raccordée à la buse,
l'orifice d'échantillonnage olfactif étant positionné sur l'extrémité arrière du tube de diffusion,
**caractérisé en ce que** le boîtier comprend un polymère biosourcé et est revêtu intérieurement d'une résine époxy à haute résistance.

2. Dispositif portable selon la revendication 1, le moyen d'insertion d'échantillon comprenant une lame d'échantillon (130) et un chariot d'échantillon (125) configuré pour recevoir la lame d'échantillon ; la lame d'échantillon étant éventuellement faite d'un matériau choisi dans le groupe constitué par le verre, le polymère, la céramique, le métal, le tissu, le papier, la peau artificielle, un échantillon de cheveux et des combinaisons de ceux-ci.

3. Dispositif portable selon la revendication 1 ou 2, le polymère biosourcé étant choisi dans le groupe constitué par l'acide polylactique, les polyhydroxyalcanoates, l'acétate de cellulose, les mélanges d'amidon et leurs combinaisons ; et le boîtier étant éventuellement entièrement détachable et lavable.

4. Dispositif portable selon l'une quelconque des revendications 1 à 3, le boîtier ayant une forme parallélépipédique ayant une largeur comprise entre environ 15 cm et environ 25 cm, une profondeur comprise entre environ 5 cm et environ 15 cm, et une hauteur comprise entre environ 5 cm et environ 15 cm.

5. Dispositif portable selon l'une quelconque des revendications 1 à 4, ledit tube de diffusion étant fabriqué à partir d'un matériau choisi dans le groupe constitué par le verre, un métal, un plastique, un polymère, un polymère biosourcé, et leurs combinaisons, et étant éventuellement revêtu de résine époxy.

6. Dispositif portable selon l'une quelconque des revendications 1 à 5, ledit tube de diffusion étant fabriqué à partir d'aluminium et revêtu d'une résine époxy.

7. Dispositif portable selon l'une quelconque des revendications 1 à 6, ledit tube de diffusion ayant une longueur comprise entre environ 50 cm et environ 150 cm et un diamètre de section compris entre environ 5 cm et environ 10 cm.

8. Dispositif portable selon l'une quelconque des revendications 1 à 7, le tube de diffusion étant équipé d'un orifice d'échantillonnage olfactif situé dans l'extrémité arrière du tube de diffusion.

9. Dispositif portable selon l'une quelconque des revendications 1 à 8, l'orifice d'échantillonnage olfactif comprenant un composant sélectionné dans le groupe constitué de capteurs, de bouchons à vis et de raccords configurés pour adapter un ou plusieurs instruments analytiques.

10. Dispositif portable selon la revendication 9, le système de ventilation étant configuré pour générer une impulsion d'air ; et éventuellement l'impulsion d'air durant entre 1 seconde et 5 secondes ; et éventuellement l'impulsion d'air ayant une vitesse d'écoulement d'air entre environ 1 km/h et environ 15 km/h.

11. Système (10) de mesure de la diffusion de parfums et de matières premières de parfums, comprenant :
un dispositif portable selon l'une quelconque des revendications 1 à 10 ;
une télécommande sans fil haute fréquence (HRF) (300) configurée pour commander un ou plusieurs composants du dispositif portable ; et
une structure de support (135) configurée pour loger le dispositif portable et la télécommande HRF.

12. Procédé (500) pour évaluer la traînée de parfums ou de matières premières de parfums en utilisant un dispositif portable selon l'une quelconque des revendications 1 à 10 ou système selon la revendication 11, le moyen d'insertion d'échantillon comprenant une lame d'échantillon (130) et un chariot d'échantillon (125) configuré pour recevoir la lame d'échantillon, le procédé comprenant les étapes suivantes :
(i) la préparation (510) d'un échantillon de parfums ou de matières premières de parfums sur la lame d'échantillon ;
(ii) la purge (505) du tube de diffusion pendant une durée d'environ 10 à environ 30 secondes, éventuellement à l'aide d'un bouton de purge (155) ;
(iii) l'introduction (515) de la lame d'échantillon contenant le parfum dans le système par le chariot d'insertion d'échantillon ;
(iv) le démarrage (520) du système de ventilation pour fournir une courte impulsion d'air à travers le tube de diffusion ;
(v) la réalisation (525) d'une évaluation d'intensité olfactive et/ou hédonique par un panel sensoriel entraîné ;
(vi) la connexion (530) de l'adaptateur de bouchons à vis d'orifice d'échantillonnage olfactif à un instrument analytique pour mesurer la quantité de molécules de parfums ou de matières premières de parfums ; et
(vii) l'analyse de données (535).

13. Procédé selon la revendication 12, environ 5 µL à environ 100 µL de parfums ou de matières premières de parfums étant déposés sur la lame d'échantillon pour évaluation.

14. Procédé selon l'une quelconque des revendications 12 ou 13, comprenant en outre le chauffage de l'échantillon d'environ 25 °C à environ 35 °C avant ou pendant l'évaluation olfactive.
